# EUROPEAN PATENT APPLICATION

(11) **EP 2 509 013 A1**
(43) Date of publication of application: **10.10.2012**
(21) Application number: 11160974.9
(22) Date of filing: 04.04.2011
(51) Int. Cl.: G06F 19/00

(54) **3D image navigation method**

(71) Applicant: Agfa Healthcare, 2640 Mortsel (BE)
(72) Inventor: Costa Teixeira, Jose, 2640, Mortsel (BE)

(57) **Abstract**

Method to navigate through a 3D image by manipulating a navigation device with an incorporated display unit in space and determining the position and orientation of the navigation device in space so as to define a viewpoint and section plane, virtually intersecting the 3D image with said plane, calculating data of a slice image representing the intersection of said 3D image and said plane and displaying said slice image on said display unit part of the navigation device.

## Description

### FIELD OF THE INVENTION

The present invention relates to medical 3D imaging. More particularly it relates to a method to navigate through medical 3D images previously acquired by an image acquisition device.

### BACKGROUND OF THE INVENTION

The need of examining the internals of patients in a non-invasive way let to the invention of several volumetric scanning modalities like MR, CT or PET. These scanners produce large volumetric data sets of a physical property measured on a fine volumetric grid superimposed on the subject under study.

In order to easily visually examine the volumetric data sets, volume rendering methods have been invented to display the volume directly in a 3D representation. Those methods include direct volume rendering, maximum intensity projection (MIP), minimum intensity projection (MinIP), average intensity projection, digital radiographiy reconstruction (DRR), double contrast barium enema simulation (DCBE) etc. These volume rendering methods enable the examiner to display, rotate, zoom and pan the volumetric data set in 3D.

This volumetric imaging provides enhanced visualization of anatomical details and facilitates the physician's observation and gives him a better inside view into the structures in the patient's body.

Examination of multi-dimensional images is also often performed on 2D slice images (sections) through the volume data set which are computed and visualized. Slices in 6 dimensions in space may be calculated. A technique for computing such slices is for example a multiplanar reformatting (MPR) technique.

To give the physician the best possible insight into the structures in the patient's body, the physician navigates through the 3D volume and evaluates the above-described slice images. Navigation tools which are presently available for user interaction with a displayed 3D image are rather complicated.

For exploring 3D data, a 3D mouse may for example be used as navigation tool. However, these devices are expensive and require a lot of effort for learning to work with the device because the user interface is not at all intuitive.

This problem has been recognized in the article 'Explore in 3D: a new virtual image navigation tool', Michael Teisler, 1 August 2006, SPIE newsroom, DOI: 10.117/2.1200607.0222.

To overcome the problem a solution has been proposed in this article which is based on the idea to mimic an ultrasound examination in which slice images of the patient's body are generated with a handheld 3D mouse. A 3D view of the full volume data set is shown on a display device together with arbitrarily positioned slice images and optional cutting planes. On a second display device a 2D view is shown with one selected slice image as 2D image. Instead of a traditional 2D mouse, a handheld 3D mouse is used as interaction device for the user. The position and orientation of the 3D mouse in space is used to define the position and orientation of a slice image or of the whole virtual volume.

Even with the above-described navigation method, navigation remains particularly difficult because the user has to manipulate the 3D mouse in space on one hand and has to follow and evaluate the effect of this manipulation on a display screen on the other hand, and these displays will usually be in different planes and orientations. Mental and physical coordination between the mouse movement in space and the displayed result of this movement is required and usually highly demanding, as the "object" being observed is considerably different from the "object" being manipulated.

It is thus an object of the present invention to provide a navigation method to navigate through previously acquired 3D images that overcomes the above-described disadvantages.

### SUMMARY OF THE INVENTION

The above-mentioned aspects are realised by a method having the specific features set out in claim 1.

Specific features for preferred embodiments of the invention are set out in the dependent claims.

The method of the present invention is advantageous over the prior art because in the prior art the position and orientation of the navigation device, i.e. the 3D mouse, in space is not coinciding with the slice which is displayed on the display device. There is still a lot of effort required from the user to coordinate the mouse manipulation with what is seen on the display device.

The navigation method of the present invention is a lot more intuitive. With the tablet manipulation of the present invention, the user is looking at an object as if he was really cutting it. The result of the virtual cutting operation is immediately seen (as a slice image) on the tablet computer by means of which he performs the virtual cutting operation. This form of manipulation does not require coordination and interpretation of the movement of the 3D device in space and the display of the slice on a display screen.

The virtual cutting plane, that is defined by position and orientation of the plane in which the navigation device (e.g. tablet computer) is positioned and the plane in which the display of the slice image is performed, are the same. No effort to coordinate the manipulation of the navigation tool with the effect of the manipulation (display of the slice image) is required.

The navigation tool is used as virtual scalpel plane cutting through the 3D volume. The image that the user sees on the screen of the tablet computer is what he would get if he were sectioning the virtual 3D object at the position and orientation he imposes. Consequentially, the user does not need additional eye-hand coordination efforts to translate the 'envisaged' movement into 'required' movement.

Further advantages and embodiments of the present invention will become apparent from the following description and associated drawing.

### BRIEF DESCRIPTION OF THE DRAWINGS

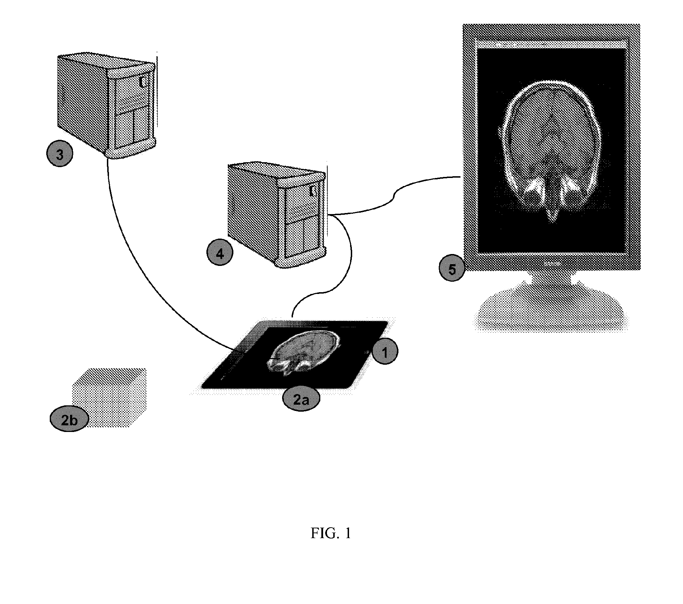

Fig. 1 shows an embodiment of a system by means of which the navigation method of the present invention can be implemented.

### DETAILED DESCRIPTION OF THE INVENTION

Figure 1 shows a system in which the method of the present invention can be implemented.

The system comprises a navigation device 1 which is in the shown embodiment implemented as a tablet computer. The navigation device is freely movable in space.

The navigation device comprises a display screen and control means to control operation of said display device and is coupled, preferably wirelessly, with a signal processor and/or a data repository. In an alternative embodiment the navigation device itself may comprise the signal processor and/or data repository.

In one embodiment the navigation device is a tablet computer. The tablet computer is freely movable in space along six degrees of freedom - translation in three perpendicular axis and rotation about three perpendicular axes.

The system further comprises a tracking system (2a, 2b) for determining the position and orientation of the plane in which the navigation device is situated when the user manipulates the navigation device in space.

The tracking system can be implemented in different ways.

In one embodiment the tracking system is a system which is able to detect the position and orientation of the navigation device relative to a reference point by simply calculating distances between certain predefined locations on the navigation device and a reference point.

In another embodiment the navigation device has sensors which can be used by a tracking device to determine the navigation device's position and orientation. Such sensors may be infrared or visible light sources, magnetic sensors, acoustic sensors, capacitive or inductive sensors, gyroscopes, accelerometers etc.

Such position detection devices are well-known in the art of computer gaming. An example of such a system is implemented in the Sixense Truemotion System.

Image data representing a digital volume representation of an image of an object are stored in a data repository 3. Said image data can be obtained in advance from various image acquisition devices which generate 3D image data such as MR, CT, PET etc.

Data repository 3 is connected to a signal processing system 4 which is capable of calculating data of slice images on the basis of the acquired 3D representation of an image which can be retrieved from data repository 3.

Techniques for calculating such slice images from a 3D data set are known in the art and comprise e.g. a multi-planar reformatting technique (MPR).

Optionally the system also comprises a display device 5 which is external to the navigation device 1 and which is arranged to receive data from processor 4 or directly (not shown) from navigation device 1.

The operation of the above-described system according to the invention is as follows.

A user moves a navigation device 1 such as a tablet computer in space (6 degrees of freedom) until the plane in which the tablet computer is situated coincides with a position and orientation of an envisaged section plane within a 3D image.

The position and orientation of the plane can be followed on a display screen on which a 3D volume representation of an object, for example a 3D skull image, is displayed.

The 3D data representing the volume image of the object, e.g. the skull, are commonly acquired earlier and are retrieved from a data repository 4. The volume representation is calculated by a signal processor 3 and the data are fed to a display device 5. Display of the 3D image is optional. However, it may be efficient when evaluating the position of the section plane and the corresponding slice image (see below).

Next, the position and orientation of the plane in which the navigation device is positioned in space and which corresponds with the section plane the user is interested in, is determined.

These coordinates defining the position and orientation of the navigation device are transmitted to signal processor 3 (which may be implemented as a separate signal processing device or which may be part of the navigation device) and data representing a slice image virtually intersecting said 3D image according to the determined plane are calculated.

A slice image corresponding with the calculated data is finally displayed on the display device part of the navigating device.

Additionally the slice image may also be displayed on an additional display device 5 which is external to the navigation device.

The navigation device may be provided with signal processing means which may be adapted to perform the calculations needed to obtain the data representing a slice image.

The image displayed in the navigation device may be of reduced quality, to improve response and/or reduce the need to send high quality images while navigating.

Additionally the signal processor in the navigation tool may be provided with image enhancing capabilities.

The navigation tool may be provided with means to pan the virtual object, i.e. using the navigation tool to "grab" the virtual object, move its orientation and position in order to further facilitate navigation.

Additionally the navigation tool or an external control connected to the navigation device (or to the tracking system or any of the system components) may be used to 'freeze' the image and to allow better observation and/or image controls (e.g. contrast and/or brightness adjustments) even if the navigation device is moved during this adjustment.

Also, the system may be used to "zoom" - expand the scale as if the expansion of an area of interest implies an equal expansion of the virtual object.

Furthermore, the system may be adapted to allow the user to "pinch and zoom", i.e. selecting at least two points in the image and drag them in the navigation tool to achieve the same effect.

The display resolution of said slice image may be adapted to benefit either visualization quality or navigation response.

## Claims

1. A method to navigate through a 3D image of an object represented by a digital signal representation comprising the steps of
- manipulating a navigation device comprising a display device in space,
- determining the position and orientation of a plane in which said navigation device is located in space,
- virtually intersecting said 3D image with said plane,
- calculating from said digital signal representation data of a slice image representing the intersection of said 3D image and said plane,
- displaying said slice image on said display device part of said navigation device.

2. A method according to claim 1 wherein said position and orientation is calculated by measuring distance(s) between (a) location(s) on said navigation device and a reference location.

3. A method according to claim 1 wherein said position and orientation is obtained by determining the position of sensors coupled to said navigation device.

4. A method according to claim 1 wherein a volume representation of said 3D image is displayed on a second display screen and wherein the intersection of said 3D image with said plane is indicated on said volume representation.

5. A method according to claim 1 wherein said navigation device comprises signal processing means arranged to calculate said data of a slice image.

6. A method according to claim 1 wherein said navigation device comprises a data repository for storing said digital signal representation.

7. A method according to claim 1 wherein said navigation device is a tablet computer.

8. A method according to claim 1 wherein the display resolution of said slice image may be adapted to benefit either visualization quality or navigation response.
